# EUROPÄISCHE PATENTSCHRIFT

(11) **EP 3 317 788 B1**
(45) Veröffentlichungstag und Bekanntmachung des Hinweises auf die Patenterteilung: **13.12.2023**
(21) Anmeldenummer: 16742184.1
(22) Anmeldetag: 04.07.2016
(51) Int. Cl.: G16H 40/20, G16H 10/60

(54) **PFLEGEUNTERSTÜTZUNGSGERÄT UND VERFAHREN ZUR PFLEGEUNTERSTÜTZUNG**
CARE SUPPORT DEVICE AND METHOD FOR CARE SUPPORT
APPAREIL D'ASSISTANCE D'ENTRETIEN ET PROCÉDÉ D'ASSISTANCE D'ENTRETIEN

(30) Priorität: 02.07.2015 DE 102015212454
(43) Veröffentlichungstag der Anmeldung: 09.05.2018
(73) Patentinhaber: Bode Chemie GmbH, 22525 Hamburg (DE)
(72) Erfinder: JAMES, Claudia, 22880 Hamburg (DE); MARCHESINI, Paolo, 89081 Ulm (DE); WITT, Nicole, 22559 Hamburg (DE)
(74) Vertreter: DREISS Patentanwälte PartG mbB
(86) Internationale Anmeldenummer: PCT/EP2016/065639
(87) Internationale Veröffentlichungsnummer: WO 2017/001699

(56) Entgegenhaltungen:
- DE-A1-102012 101 152

## Beschreibung

Die Erfindung betrifft ein Pflegeunterstützungsgerät und ein Verfahren zur Pflegeunterstützung.

Bei der Pflege von Patienten, insbesondere bei der Durchführung von invasiven oder minimal-invasiven Maßnahmen oder bei Handlungen die der Wundversorgung oder der intravenösen Verabreichung von Medikamenten oder deren Vorbereitung dienen, ist die Beachtung hygienischer Standards unerlässlich. Arbeitsabläufe, die jeweils erforderliche hygienische Maßnahmen in der richtigen Reihenfolge angeben, müssen daher regelmäßig vom Fachpersonal eingehalten werden.

Zudem können einzelne Arbeitsschritte unterschiedlich wichtige Bedeutung bezüglich der Pflege haben.

DE 10 2012 101 152 A1 offenbart eine Fitnessvorrichtung die berührungslos Bewegungen eines Benutzers erfasst, ein Abbild des Benutzers generiert, Bewegungsabläufe dieses Abbilds mit hinterlegten Fitnessübungen vergleicht, Abweichungen feststellt und auf einem Display überlagert darstellt.

Beispielsweise sind infektionsrelevante Schritte, die mit Fokus auf Patientenschutz und die Vermeidung von therapieassoziierten Infektionen (HAI) unerlässlich sind, in diesbezüglichen Anweisungen oder Empfehlungen festgelegt. Derartige Empfehlungen werden beispielsweise von der World Health Organisation (WHO), einem Center for Desease Control (CDC) oder dem Robert-Koch-Institut (RKI) herausgegeben.

Beispielsweise werden diese Empfehlungen in Kategorien in einer Richtlinie für Krankenhaushygiene und Infektionsprävention angegeben. Bekannt sind z.B. die Kategorien I A, I B, ..., die in ihrer aktuellen Version beispielsweise vom RKI oder einem CDC veröffentlicht werden.

In Arbeitsabläufen müssen diese Empfehlungen bei der Durchführung der Pflege eingehalten werden. Diese können beispielsweise auch in Lehrbüchern veröffentlicht sein. Dadurch soll eine bestmögliche Pflege eines Patienten gewährleistet werden.

Um dies zu gewährleisten werden Pflegekräfte entsprechend aus- und weitergebildet.

Allerdings entsteht bei komplexen Arbeitsabläufen, aufgrund neuer wissenschaftlicher Erkenntnisse oder neuer medizinischer Apparaturen oder Hilfsmittel die Problematik, dass sich Arbeitsabläufe ändern. Arbeitsschritte, die fester Bestandteil eines Arbeitsablaufs sind, müssen dadurch beispielsweise ersetzt, an einer anderen Stelle des Arbeitsablaufs oder mit anderen Hilfsmitteln erfolgen.

Zur Gewährleistung von Qualitätsstandards kann die korrekte Durchführung der Arbeitsabläufe in Krankenhäusern von Beobachtern dokumentiert oder überprüft werden.

Ein Beobachter steht dabei zunehmend vor der Herausforderung, die korrekte Durchführung der komplexen Arbeitsabläufe, in Echtzeit zu erfassen. Dazu muss der Beobachter eine Vielzahl sich ändernder komplexer Arbeitsabläufe in derselben, teilweise sehr hohen Geschwindigkeit, in der die einzelnen Arbeitsschritte durchgeführt werden müssen, erfassen, bewerten oder beurteilen.

Ein Nachschlagen in einem Lehrbuch oder mehreren Lehrbüchern kommt dazu aus Zeitgründen nicht in Frage, da die Reihenfolge der Arbeitsschritte zueinander, die Schwere der Auswirkungen einer fehlerhaften Reihenfolge oder des Auslassens eines Arbeitsschritts aufgrund der Komplexität und der sich ändernden Arbeitsabläufe dadurch nicht in Echtzeit möglich ist.

Der vorliegenden Erfindung liegt die Aufgabe zugrunde ein Pflegeunterstützungsgerät und ein Verfahren zur Pflegeunterstützung zu schaffen, das die bestehenden Einschränkungen bei der Beobachtung von Arbeitsabläufen in der Pflege beseitigt.

Diese Aufgabe wird durch das Pflegeunterstützungsgerät nach Anspruch 1 gelöst.

Diese Aufgabe wird ebenfalls mit dem Verfahren zur Pflegeunterstützung nach Anspruch 5 gelöst.

Dadurch wird dem menschlichen Beobachter die Wahrnehmung und Aufnahme von Information in Echtzeit ermöglicht, die die Erfassung, Bewertung oder Beurteilung des Pflegeablaufs in Echtzeit zweckmäßig gestaltet oder für bestimmte, insbesondere komplexe Arbeitsabläufe erst ermöglicht.

Weitere Einzelheiten, Merkmale und Vorteile der Erfindung ergeben sich aus den beigefügten Patentansprüchen und aus der zeichnerischen Darstellung und nachfolgenden Beschreibung bevorzugter Ausführungsformen der Erfindung. In der Zeichnung zeigt:
Figur 1 schematisch einen Teil einer Ausführungsform des erfindungsgemäßen Geräts;
Figur 2 schematisch eine Darstellung eines Soll-Pflegeablaufs;
Figur 3 schematisch eine Darstellung eines Ist-Pflegeablaufs;
Figur 4 schematisch eine Darstellung eines Soll-Pflegeablaufs und eines Ist-Pflegeablaufs gleichzeitig, nebeneinander;
Figur 5 schematisch eine Benutzeroberfläche.

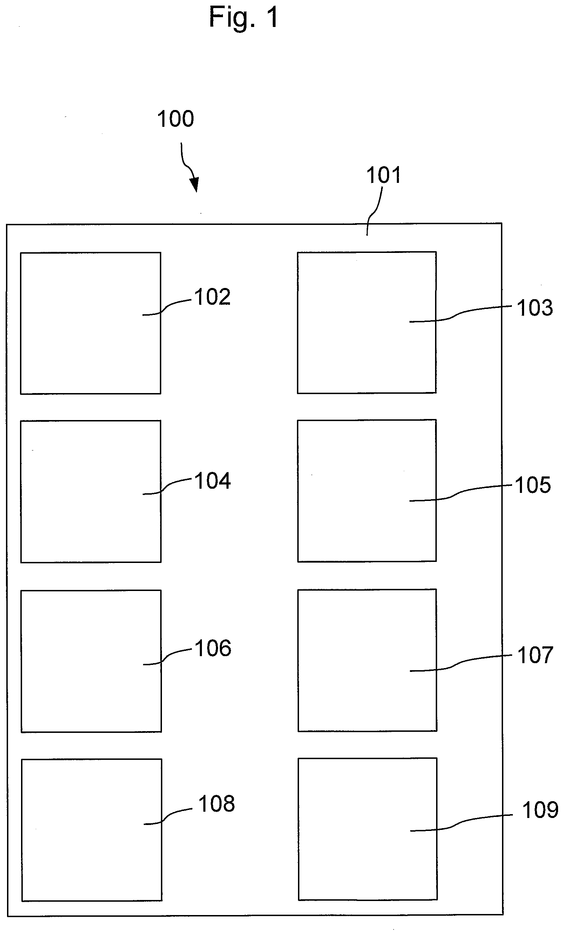

In Figur 1 ist ein Pflegeunterstützungsgerät 100 dargestellt.

Das Pflegeunterstützungsgerät 100 ist beispielsweise ein tragbarer flacher Rechner.

Das Pflegeunterstützungsgerät 100 weist eine Bildausgabeeinrichtung 102 auf. Die Bildausgabeeinrichtung 102 ist dazu eingerichtet Symbole darzustellen, die einen Pflegeablauf visualisieren. Die Bildausgabeeinrichtung 102 weist im Beispiel einen berührungsempfindlichen Bildschirm 101 zur Darstellung der Symbole auf. Die Bildausgabeeinrichtung 102 umfasst beispielsweise eine Grafikkarte (nicht dargestellt) durch die der Bildschirm 101 zur Ausgabe der Symbole angesteuert wird.

Das Pflegeunterstützungsgerät 100 weist eine Vorgabeeinrichtung 103 auf, die dazu eingerichtet ist, darzustellende Symbole basierend auf einem Soll-Pflegeablauf vorzugeben. Die Symbole werden im Beispiel der Bildausgabeeinrichtung 102 vorgegeben und durch diese in einem jeweils durch die Reihenfolge der Vorgabe festgelegten Bereich auf dem berührungsempfindlichen Bildschirm 101 ausgegeben. Dazu wird im Beispiel ein Symbol das den jeweiligen Arbeitsschritt eines Pflegeablaufs visualisiert im festgelegten Bereich in dem das jeweilige Symbol angezeigt wird angeordnet

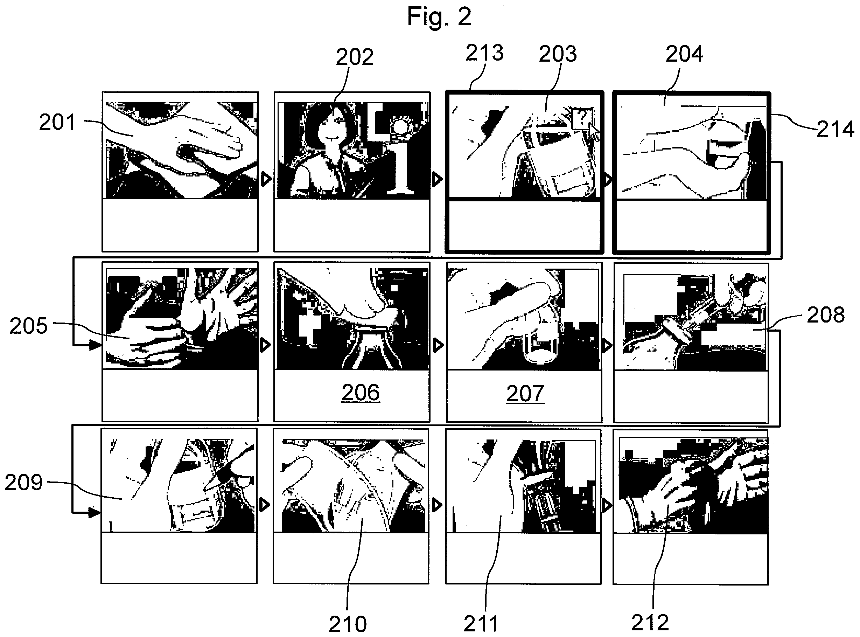

Eine Ausgabe von Symbolen 201 - 212 eines derartigen Soll-Pflegeablaufs durch die Bildausgabeeinrichtung 102 ist in Figur 2 schematisch dargestellt. Die erste Reihenfolge der Vorgabe und die Reihenfolge der Wiedergabe stimmen im Beispiel überein. Die Reihenfolge ist durch kleine Pfeile startend beim Symbol 201 dargestellt. Dies erleichtert die Wahrnehmung des Soll-Pflegeablaufs.

Bevorzugt ist vorgesehen, besonders wichtige Schritte mit einem Rand hervorzuheben. Beispielsweise wird das Symbol 203 mit einem ersten Rand 213 dargestellt. Das Symbol 204 wird beispielsweise mit einem zweiten Rand 214 dargestellt. Vorzugsweise unterscheiden sich die Ränder durch ihre Form oder Farbe.

Das Pflegeunterstützungsgerät 100 weist eine Erfassungseinrichtung 104 auf, die dazu eingerichtet ist, eine Benutzereingabe zu erfassen und dargestellte Symbole basierend auf der Benutzereingabe einem Ist-Pflegeablauf zuzuordnen. Die Erfassungseinrichtung 104 wirkt im Beispiel mit dem berührungsempfindlichen Bildschirm 101 zusammen. Die Erfassungseinrichtung 104 erkennt eine Berührung eines Benutzers und deren Position auf dem Bildschirm 101. Die Erfassungseinrichtung 104 ordnet die erkannte Position dem Ist-Pflegeablauf zu, beispielsweise indem eine neue Position in einer zweiten Reihenfolge des Ist-Pflegeablaufs hinten angefügt wird. Das bedeutet, dass durch die zweite Reihenfolge das Symbol, das in dem festgelegten Bereich dargestellt wird, der mit der erkannten Position überlappt in der zweiten Reihenfolge dem Ist-Pflegeablauf zugeordnet wird. Das bedeutet, dass der jeweilige dem Symbol zugeordnete Arbeitsschritt dem Ist-Pflegeablauf zugeordnet wird.

Das Pflegeunterstützungsgerät 100 weist zudem eine Bilderzeugungseinrichtung 105 auf, die dazu eingerichtet ist, Symbole, die den Ist-Pflegeablauf visualisieren, zu erzeugen. Dazu wird im Beispiel zusätzlich zu einem Symbol das den jeweiligen Arbeitsschritt eines Pflegeablaufs visualisiert, Information zur zweiten Reihenfolge im Bereich, in dem das jeweilige Symbol angezeigt wird, angeordnet.

Dazu ist die Bilderzeugungseinrichtung 105 eingerichtet, basierend auf der erfassten Benutzereingabe, mindestens einem dargestellten Symbol, das dem Soll-Pflegeablauf zugeordnet ist, ein vorgegebenes Symbol, das dem Ist-Pflegeablauf zugeordnet ist, zu überlagern.

Beispielsweise werden die Symbole, die die jeweiligen Arbeitsschritte eines Pflegeablaufs visualisieren, mit der jeweiligen Information zur Reihenfolge im Bereich in dem das jeweilige Symbol angezeigt wird überlagert.

Alternativ kann das Symbol, das den Arbeitsschritt des Soll-Pflegeverlaufs darstellt, durch ein Symbol das den Ist-Pflegeablauf darstellt ersetzt werden. Dazu können zusätzlich zu den Symbolen die Arbeitsschritte des Soll-Pflegeablaufs darstellen, weitere Symbole vorgesehen sein, die den identischen Arbeitsschritt darstellen und die Information zur zweiten Reihenfolge aufweisen.

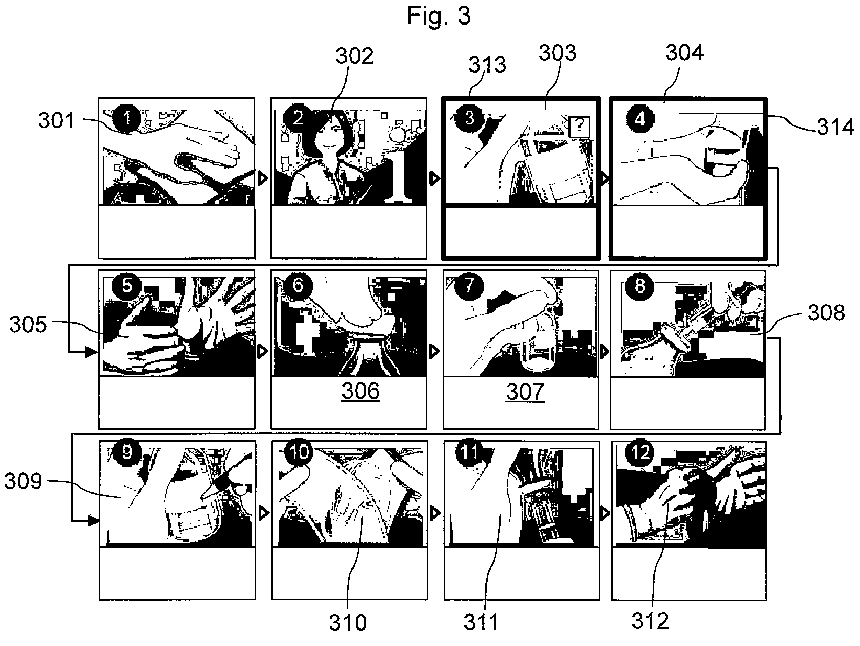

Eine Ausgabe von Symbolen 301 - 312 eines Ist-Pflegeablaufs durch die Bildausgabeeinrichtung 102 ist in Figur 3 schematisch dargestellt. Die erste Reihenfolge des Soll-Pflegeablaufs und die zweite Reihenfolge des Ist-Pflegeablaufs stimmen im Beispiel überein. Die zweite Reihenfolge ist im Beispiel durch kleine Nummern in Kreisen, startend bei Nummer 1 beim Symbol 301 und endend bei Nummer 12 beim Symbol 312 dargestellt. Diese Nummern sind im Beispiel in einer Ecke des jeweiligen Symbols angeordnet. Dies erleichtert die Wahrnehmung des Ist-Pflegeablaufs. Jede andere Symbolik als arabische Zahlen, beispielsweise römische Zahlen können ebenfalls verwendet werden. Die Position an der die Nummer dargestellt wird kann auch in einem anderen Teil des festgelegten Bereichs des jeweiligen Arbeitsschritts liegen, beispielsweise in der Mitte.

Bevorzugt ist vorgesehen, besonders wichtige Schritte mit einem Rand hervorzuheben. Beispielsweise wird das Symbol 303 mit einem ersten Rand 313 dargestellt. Das Symbol 312 wird beispielsweise mit einem zweiten Rand 314 dargestellt. Vorzugsweise wird die Darstellung des Randes entsprechend der Darstellung des Randes für das Symbol des Soll-Pflegeablaufs vorgegeben.

Das Pflegeunterstützungsgerät 100 weist zudem eine Vergleichseinrichtung 106 auf, die dazu eingerichtet ist, die erste Reihenfolge, in der die darzustellenden Symbole basierend auf dem Soll-Pflegeablauf vorgegeben werden, mit der zweiten Reihenfolge zu vergleichen, in der Benutzereingaben zu den dargestellten Symbolen erfasst wurden. Beispielsweise wird dazu zu jeder bei einer Benutzereingabe erfassten Position aus der zweiten Reihenfolge das Symbol in der ersten Reihenfolge gesucht das an dieser Position angezeigt wurde. Dazu wird beispielsweise geprüft, ob der festgelegte Bereich an dem das Symbol angezeigt wurde mit der Position überlappt. Anschließend wird überprüft, ob sich die jeweiligen Einträge an derselben Stelle der jeweiligen Reihenfolge befinden.

Die Bildausgabeeinrichtung 105 ist ausgebildet gleichzeitig Symbole, die den Soll-Pflegeablauf visualisieren und Symbole die den Ist-Pflegeablauf visualisieren, darzustellen.

Symbole, die einzelne Arbeitsschritte visualisieren sind beispielsweise in einem Speicher (nicht dargestellt) im Pflegeunterstützungsgerät 100 abgespeichert. Diese Symbole werden beispielsweise aus dem Speicher gelesen und in der entsprechenden ersten Reihenfolge vorgegeben.

Das Pflegeunterstützungsgerät 100 weist ferner eine Auswerteeinrichtung 107 auf, die dazu eingerichtet ist, eine Spontanbewertung vorzugeben. Die Auswerteeinrichtung 107 erzeugt dazu abhängig vom Ergebnis des Vergleichs der ersten Reihenfolge des Soll-Pflegeablaufs mit der zweiten Reihenfolge des Ist-Pflegeablaufs Information zur Bewertung. Im Beispiel werden als Spontanbewertung Emoticons, insbesondere die schematische Darstellung eines lachenden, eines neutralen oder eines unzufriedenen Gesichts verwendet. Diese können bevorzugt in den Farben Grün, Orange bzw. Rot gefärbt sein.

Bei Übereinstimmung der Stellen eines Arbeitsschritts in den beiden Reihenfolgen wird beispielsweise ein grünes oder lachendes Gesicht als Spontanbewertung vorgegeben. Anderenfalls wird beispielsweise ein rotes oder unzufriedenes Gesicht als Spontanbewertung vorgegeben.

Die Auswerteeinrichtung 107 kann ausgebildet sein, bei Übereinstimmung oder fehlender Übereinstimmung der Stellen eines Arbeitsschritts in den beiden Reihenfolgen eine weitere Fallunterscheidung zur Spontanbewertung zu berücksichtigen. Beispielsweise wird je nach der Bedeutung des Arbeitsschritts für die Hygiene entweder ein rotes oder unzufriedenes Gesicht bzw. ein oranges oder neutrales Gesicht angezeigt. Zu dieser Fallunterscheidung werden beispielsweise die Kategorien, insbesondere I A bzw. I B, des RKI oder eines CDC verwendet. Denkbar ist auch, zu dieser Fallunterscheidung alternativ oder zusätzlich weitere Kategorien infektionsschutzrelevanter Arbeitsschritte (Z, Y, X, ...) zu verwenden.

Bevorzugt wird eine Spontanbewertung für Arbeitsschritte angezeigt, die einer derartigen Kategorie zugeordnet sind.

Die Bildausgabeeinrichtung 105 zeigt dann die Spontanbewertung gleichzeitig mit den Symbolen, die den Soll-Pflegeablauf und den Ist-Pflegeablauf visualisieren.

Daher ist die Bildausgabeeinrichtung 105 eingerichtet, die vorgegebene Spontanbewertung benachbart zu dem jeweiligen zugeordneten Symbol oder dem jeweiligen zugeordneten Symbol überlagert darzustellen.

Das Pflegeunterstützungsgerät 100 weist zudem optional eine Eingabeeinrichtung 108 auf, die dazu eingerichtet ist, basierend auf der erfassten Benutzereingabe einen Eingabedialog vorzugeben, wobei die Bildausgabeeinrichtung 105 den Eingabedialog gleichzeitig mit, insbesondere benachbart zu den Symbolen, die den Soll-Pflegeablauf und den Ist-Pflegeablauf visualisieren, anzeigen kann. Dieser Eingabedialog umfasst beispielsweise Bemerkungen oder Anmerkungen zu einem Arbeitsschritt. Die Zuordnung erfolgt beispielsweise mittels einer für den jeweiligen Arbeitsschritt vorgesehenen Liste, die vorgegebene Bemerkungen oder Anmerkungen enthält. Diese sind beispielsweise in der Eingabeeinrichtung 108 gespeichert. Diese können beispielsweise in dem Eingabedialog als Liste bzw. Checkliste angezeigt werden.

Der Eingabedialog umfasst beispielsweise je auswählbarer Bemerkung oder Anmerkung einen zugeordneten Auswahlknopf. Eine Benutzereingabe beispielsweise eine Berührung des berührungsempfindlichen Bildschirms 101 im Bereich der Anzeige des Auswahlknopfs wird von der Eingabeeinrichtung 108 erfasst und als Auswahl der jeweiligen Bemerkung oder Anmerkung interpretiert. Die jeweilige Bemerkung oder Anmerkung wird beispielsweise zusammen mit Information über den Arbeitsschritt dem der Eingabedialog zugeordnet ist abgespeichert.

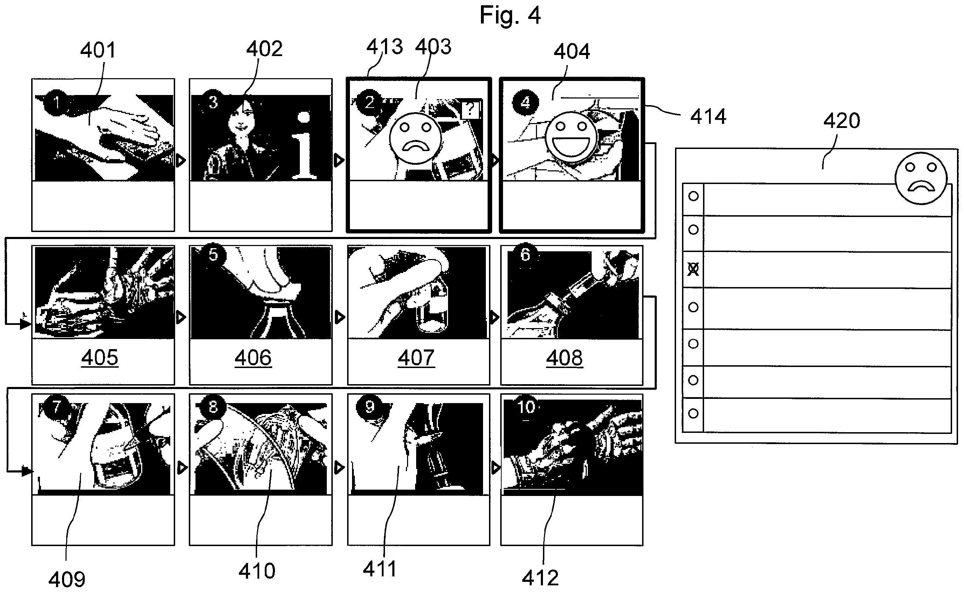

Eine Ausgabe von Symbolen 401 - 412 eines Ist-Pflegeablaufs durch die Bildausgabeeinrichtung 102 ist in Figur 4 schematisch dargestellt. Die erste Reihenfolge des Soll-Pflegeablaufs und die zweite Reihenfolge des Ist-Pflegeablaufs stimmen im Beispiel nicht überein. Die Reihenfolge der Benutzereingabe ist im Beispiel wie für Figur 3 beschrieben durch kleine Nummern in Kreisen dargestellt. Aufgrund der Benutzereingabe ergibt sich der in Tabelle 1 angegebene Vergleich der ersten Reihenfolge mit der zweiten Reihenfolge. In Tabelle 1 wird die erste Reihenfolge durch die Nummerierung der Symbole der Arbeitsschritte in Figur 4 wiedergegeben. Die zweite Reihenfolge wird mit den in Figur 4 dargestellten Nummern im Kreis wiedergegeben. Andere Zuordnungen beispielsweise durch Namen sind ebenfalls möglich.

Beispielsweise sind die Arbeitsschritte durch einen Titel, der unterhalb der graphischen Symbole eingeblendet wird erläutert. Im Beispiel wird ein Arbeitsablauf "Mischfusion zubereiten" Symbolisch dargestellt. Zudem kann unter den Symbolen in den Figuren 2 bis 4 folgendes angezeigt werden:
201, 301, 401: Arbeitsfläche wischdesinfiziert
202, 302, 402: Materialien zusammengestellt
203, 303, 403: Infusion-/Arzneimittellösung kontrolliert
204, 304, 404: Hände desinfiziert
205, 305, 405: Antibiotika Zytostatika: Handschuhe angezogen
208, 306, 406: Gummisepten der Flaschen desinfiziert
207, 307, 407: Arzneimittel gelöst
208, 308, 408: Arzneimittel der Infusionslösung beigegeben
209, 309, 409: Infusionsflasche beschriftet
210, 310, 410: Infusionssystem ausgepackt
211, 311, 411: Infusionssystem mit Infusionsflasche verbunden
212, 312, 412: Ggf. Handschuhe ausgezogen

Bevorzugt ist vorgesehen, besonders wichtige Schritte mit einem Rand hervorzuheben. Beispielsweise wird das Symbol 403 mit einem ersten Rand 413 dargestellt. Das Symbol 404 wird beispielsweise mit einem zweiten Rand 414 dargestellt. Vorzugsweise wird dieselbe Darstellung der Ränder verwendet, die auch bei der Darstellung des Soll-Pflegeablaufs vorgegeben wurde.

Die Darstellung eines der zuvor beschriebenen Ränder wird beispielsweise basierend auf den Empfehlungen von WHO, RKI oder CDC gewählt, beispielsweise abhängig von der Kategorie. Beispielsweise wird die Randdarstellung für Kategorie I A unterschiedlich von der Darstellung für Kategorie I B vorgegeben. Symbole, die keiner Kategorie entsprechen werden im Beispiel ohne Rand dargestellt. Als Unterschied wird beispielsweise ein Rand eines Symbols der Kategorie I A dicker oder rot dargestellt. Beispielsweise wird der Rand eines Symbols der Kategorie I B dünner oder gelb dargestellt.

Es kann aber auch vorteilhaft sein, die Symbole der Kategorien IA und IB oder weiterer Kategorien (Z, Y, X, ...) gleichartig, beispielsweise jeweils mit einem dicken oder roten Rand hervorzuheben.

Die Information über den Soll- und Ist-Pflegeverlauf sowie die beschriebenen zusätzliche Information zu Kategorie oder zum Eingabedialog ist in folgender Tabelle 1 dargestellt. Diese kann beispielsweise in dem Pflegeunterstützungsgerät erstellt, gespeichert und geändert werden.

**Tabelle 1**

| erste Reihenfolge | zweite Reihenfolge | Kategorie | Eingabedialog |
|---|---|---|---|
| 401 | 1 | - | |
| 402 | 3 | - | |
| 403 | 2 | Z | Bemerkung 1 |
| 404 | 4 | Y | |
| 405 | - | - | |
| 406 | 5 | - | |
| 407 | - | - | |
| 408 | 6 | - | |
| 409 | 7 | - | |
| 410 | 8 | - | |
| 411 | 9 | - | |
| 412 | 10 | - | |

Dem Ergebnis des Vergleichs der Reihenfolgen entsprechend, wurde die Reihenfolge der Schritte 2 und 3 vertauscht. Dies wird für den Beobachter durch die Angabe der Nummern im Kreis im Bereich der Anzeige der betroffenen Arbeitsschritte sofort ersichtlich. Die Spontanbewertung ist in Figur 4 im Bereich der Anzeige der Symbole 403 und 404 dargestellt. Diese Symbole betreffen Arbeitsschritte, die den Kategorien infektionsschutzrelevanter Arbeitsschritte Z, Y zugeordnet sind. Nicht in der zweiten Reihenfolge enthaltene Arbeitsschritte der ersten Reihenfolge erhalten im Beispiel keine Nummerierung. Dadurch wird ersichtlich, welche Arbeitsschritte nicht durchgeführt wurden. Soll- und Ist-Pflegeablauf werden somit gleichzeitig nebeneinander dargestellt. Dies erleichtert die Wahrnehmung des Ist-Pflegeablaufs und der Unterschiede zwischen dem Soll- und dem Ist-Pflegeablauf.

Außerdem wird aufgrund des Vergleichsergebnisses ein Eingabedialog 420 angezeigt. Der Eingabedialog 420 ermöglicht im Beispiel zu dem Arbeitsschritt der als Symbol 403 dargestellt wurde, eine Bemerkung 1 einzugeben. Der Eingabedialog wird beispielsweise beim Hinzufügen des Arbeitsschritts zur zweiten Reihenfolge ausgegeben. Beispielsweise wird der Eingabedialog beim Erkennen einer Benutzereingabe ausgegeben, durch die das Symbol, das diesen Arbeitsschritt darstellt, ausgewählt wird.

Die Bemerkung 1 wird beispielsweise aus der Liste vorgegebener Bemerkungen wie beschrieben ausgewählt.

Im Beispiel wird ein Aseptik Dialog angezeigt bestehend aus den Punkten:
- Septum wurde nach Desinfektion berührt
- Metalldorn von Kanüle wurde berührt,
- Die Spritzenöffnung wurde berührt,
- Für Lösen und Beigeben von Arzneimittel wurde die gleiche Spritze verwendet,
- Septum wurde mit bereits verwendeten Kanüle durchstochen,
- Der Einstichdorn des Infusionssystems wurde berührt,
- Spritze Kanüle, Infusionssystem waren nicht steril.

Eine erkannte Benutzereingabe wird beispielsweise in Form des Texts der Liste als Bemerkung der Tabelle 1 abgespeichert. Dies erfolgt beispielsweise sobald mindestens ein Eintrag aus der Liste ausgewählt wurde. Dadurch werden zusätzliche Teilschritte eines Arbeitsschritts beobachtbar und erfassbar.

Anstelle der oben angegebenen Liste kann auch eine Frage mit einer Liste von Antworten ausgegeben werden. Beispielsweise wird dann als Bemerkung die Antwort(en) gespeichert.

Besonders bevorzugt ist die Zuordnung der Arbeitsschritte zu den Kategorien, beispielsweise in Tabelle 1, derart vorgegeben, dass der Benutzer die Kategorien nicht selbst festlegen kann.

Besonders bevorzugt ist das Pflegeunterstützungsgerät 100 ausgebildet, innerhalb eines bestimmten Zeitraums nach einer erkannten Benutzereingabe die Spontanbewertung oder die Information zur Reihenfolge anzuzeigen. Der Zeitraum beträgt beispielsweise 1 Millisekunde bis 10 Sekunden, vorzugsweise 1 Millisekunde bis 5 Sekunden, 1 Millisekunde bis 1 Sekunde insbesondere weniger als 100 Millisekunden. Es kann vorgesehen sein, ein separates Symbol zur Auslösung der Bewertung vorzusehen. Eine Bewertung aller Arbeitsschritte wird dann erst nach dem Erfassen einer Benutzereingabe durch die dieses Symbol ausgewählt wurde ausgelöst.

Optional kann das Pflegeunterstützungsgerät 100 eine Kommunikationsschnittstelle 109 umfassen, die eingerichtet ist, Information über den Ist-Pflegeablauf insbesondere adressiert an einen Server zu senden. Dieser Vorgang kann durch die Auswahl eines entsprechenden Symbols ausgelöst werden.

Der Server ist beispielsweise ausgebildet, die Information mehrerer Pflegeunterstützungsgeräte 100 zu empfangen.

Besonders bevorzugt ist es, die Information zur Spontanbewertung oder dem Ist-Pflegeablauf derart anzuzeigen, dass eine Bildschirmansicht nicht speicherbar ist.

Beispielsweise wird die in Tabelle 1 angegebene Information mittels einer Internetverbindung übertragen. Dazu wird beispielsweise eine Internet Protocol / Transmission Control Protokoll Verbindung zwischen dem Pflegeunterstützungsgerät 100 und dem Server aufgebaut. Die Information wird beispielsweise mittels des Hypertext Transfer Protokolls oder des Hypertext Transfer Protokoll Secure übertragen.

Nach einer ersten Variante umfassen die an den Server übertragenen Informationen sämtliche Informationen zu dem Ist-Pflegeablauf. Insbesondere ist aber vorgesehen, dass die an den Server übertragenen Informationen nur einen Ausschnitt mithin eine Teilmenge der Informationen zu dem Ist-Pflegeablauf umfassen. So enthalten die an den Server übertragenen Informationen insbesondere Informationen zu den Arbeitsschritten, die den Kategorien infektionsschutzrelevanter Arbeitsschritte zugeordnet sind. Es kann vorzugsweise vorgesehen sein, dass die an den Server übertragenen Informationen insbesondere keine Informationen umfassen zu den Arbeitsschritten, die keiner Kategorie infektionsschutzrelevanter Arbeitsschritte zugeordnet sind.

Im Beispiel ist das Pflegeunterstützungsgerät 100 als Tablet Computer dargestellt. Die einzelnen zuvor beschriebenen Komponenten sind in diesem enthalten. Beispielsweise weist das Pflegeunterstützungsgerät 100 einen oder mehrere Mikroprozessoren auf, die einen Programmcode ausführen können, der das im Folgenden beschriebene Verfahren ausführt, wenn der Programmcode auf dem Tablet abläuft. Der Programmcode ist beispielsweise in einem nicht dargestellten Speicher im Tablet gespeichert.

Die Pflegeunterstützungsgeräte 100 übertragen beispielsweise eine eindeutige Identifikation, z.B. ihre Internet Protokoll Adresse oder einen Namen, an den Server um eine Zuordnung der übertragenen Daten zu dem jeweiligen Pflegeunterstützungsgerät 100 zu ermöglichen. Der Name ist beispielsweise der Name der Station in einem bestimmten Krankenhaus oder der Name des Krankenhauses, das das jeweilige Pflegeunterstützungsgerät 100 einsetzt. Der Server behält diese Zuordnung bei oder erzeugt eine Zuordnung mittels der eindeutigen Identifikation.

Diese Information zum Pflegeunterstützungsgerät 100 ist beispielsweise im Pflegeunterstützungsgerät 100 abgespeichert. Bevorzugt umfasst das Pflegeunterstützungsgerät 100 eine graphische Benutzerschnittstelle, mittels der der Name vor Beginn der Beobachtung eingegeben werden kann.

Vorzugsweise werden die Informationen zu mehreren Beobachtungen, beispielsweise aus mehreren Tabellen, die von einem bestimmten Pflegeunterstützungsgerät 100 empfangen werden, konsolidiert. Das bedeutet, dass beispielsweise ein Mittelwert über das Ergebnis des Vergleichs der Reihenfolgen für einen bestimmten Arbeitsablauf gespeichert wird. Alternativ oder zusätzlich kann auch eine Standardabweichung oder ein prozentuale Angabe der erkannten Abweichungen oder Übereinstimmungen verwendet werden.

Die Konsolidierung kann auch vor dem Versenden in dem Pflegeunterstützungsgerät 100 stattfinden. In diesem Fall werden beispielsweise aus mehreren Beobachtungen entstandene Tabellen, die wie die Tabelle 1 aufgebaut sind, eine konsolidierte Tabelle erzeugt und übertragen, die wie die Tabelle 1 aufgebaut ist.

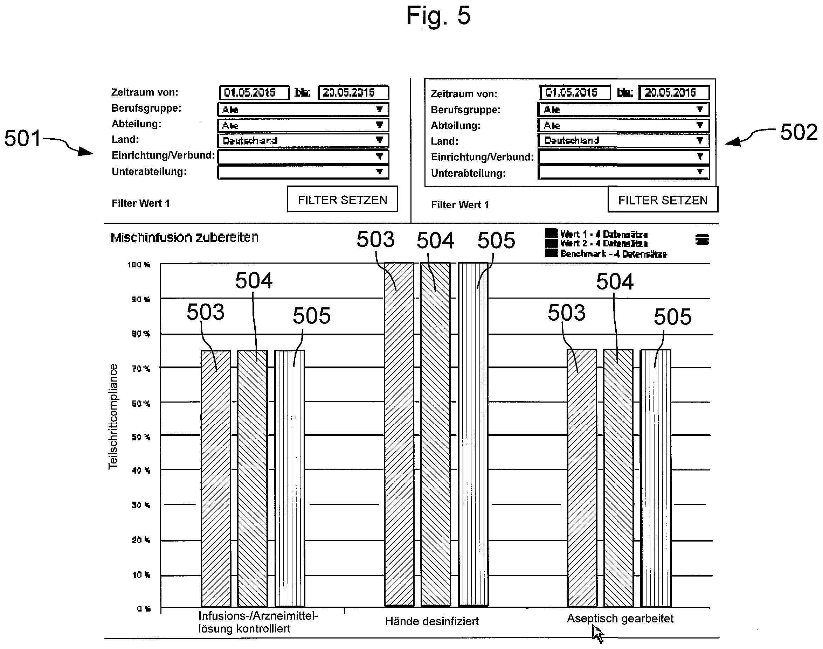

Der Server ist ausgebildet die Daten aus den Tabellen verschiedener Pflegeunterstützungsgeräte 100 oder verschiedener Krankenhäuser nebeneinander darzustellen. Eine entsprechende Oberfläche 500 ist in Figur 5 dargestellt.

Beispielsweise ist eine erste Eingabemaske 501 zur Auswahl einer ersten Station oder eines ersten Krankenhauses vorgesehen. Beispielsweise ist eine zweite Eingabemaske 502 zur Auswahl einer zweiten Station oder eines zweiten Krankenhauses vorgesehen.

Dargestellt werden beispielsweise in einem ersten Balken 503 und einem zweiten Balken 504 für einen vorgegebenen oder wählbaren Arbeitsablauf oder einen vorgegebenen oder wählbaren Arbeitsschritt die Vergleichsergebnisse der in der Eingabemaske gewählten Stationen oder Krankenhäuser. Zudem kann ein Vergleich mit einem Vergleichswert 505 vorgesehen sein.

Im Folgenden wird anhand der Darstellungen in der Figur 4 ein Verfahren zur Pflegeunterstützung beschrieben. Das Verfahren startet beispielsweise, wenn Programmcode, beispielsweise eine Applikation auf dem Tablet gestartet wird.

Nach dem Start wird optional ein erster Startbildschirm dargestellt. Der erste Startbildschirm stellt eine Benutzerschnittstelle zur Verfügung. Die Benutzerschnittstelle ermöglicht beispielsweise die Auswahl eines Namens für die Station oder das Krankenhaus des Beobachters oder die Auswahl eines zu beobachtenden Arbeitsablaufs oder einer Berufsgruppe.

Diese Information wird beispielsweise verwendet, um einen Datensatz umfassend die eingegebene Information anzulegen. Der Datensatz wird im Beispiel einer Tabelle zugeordnet, die wie die beschriebene Tabelle 1 aufgebaut ist.

Im nächsten Schritt werden darzustellende Symbole basierend auf einem vorgegebenen oder dem ausgewählten Soll-Pflegeablauf vorgegeben. Beispielsweise werden die Symbole in der ersten Reihenfolge vorgegeben.

Anschließend werden die Symbole, die den ausgewählten Soll-Pflegeablauf visualisieren, auf der Bildausgabeeinrichtung 102 angezeigt. Beispielsweise werden die Symbole in der ersten Reihenfolge angezeigt.

Dann wird eine Benutzereingabe erfasst. Dazu wird beispielsweise die Position der Benutzereingabe auf dem berührungsempfindlichen Bildschirm erfasst.

Im folgenden Schritt wird ein dargestelltes Symbol basierend auf der Benutzereingabe zum Ist-Pflegeablauf zugeordnet. Beispielsweise wird die erfasste Position an die zweite Reihenfolge angehängt.

Anschließend wird ein Symbol, das den Ist-Pflegeablauf visualisiert, erzeugt. Beispielsweise wird die darzustellende Nummer anhand der Stelle an der die erfasste Position in der zweiten Reihenfolge steht ermittelt.

In einem weiteren Schritt wird die erste Reihenfolge mit der zweiten Reihenfolge verglichen.

Dann wird auf der Bildausgabeeinrichtung gleichzeitig mit mindestens einem Symbol, das den Soll-Pflegeablauf visualisiert, das Symbol dargestellt, das den Ist-Pflegeablauf visualisiert.

Dann wird dazu basierend auf der erfassten Benutzereingabe mindestens ein dargestelltes Symbol, das dem Soll-Pflegeablauf zugeordnet ist, durch mindestens ein vorgegebenes Symbol, das dem Ist-Pflegeablauf zugeordnet ist, überlagert.

Bevorzugt wird, insbesondere falls dies für den jeweiligen Arbeitsschritt vorgesehen ist, der Eingabedialog basierend auf der erfassten Benutzereingabe vorgegeben und gleichzeitig mit, insbesondere benachbart zu den Symbolen, die den Soll-Pflegeablauf und den Ist-Pflegeablauf visualisieren, angezeigt.

Bevorzugt erfolgt die gleichzeitige Darstellung innerhalb des bestimmten Zeitraums nach der erkannten Benutzereingabe.

Es wird ferner, insbesondere falls dies für den jeweiligen Arbeitsschritt vorgesehen ist, die Spontanbewertung vorgegeben und gleichzeitig mit den Symbolen, die den Soll-Pflegeablauf und den Ist-Pflegeablauf visualisieren angezeigt.

Die vorgegebene Spontanbewertung wird einem bestimmten dargestellten Symbol zugeordnet, und benachbart zu dem jeweiligen zugeordneten Symbol oder dem jeweiligen zugeordneten Symbol überlagert dargestellt.

Diese Schritte werden bevorzugt wiederholt, insbesondere bis eine Benutzereingabe erkannt wird, die den Abschluss der Beobachtung signalisiert. Dadurch wird der Ist-Pflegeablauf gleichzeitig und insbesondere neben dem Soll-Pflegeablauf dargestellt.

Optional kann Information über den Ist-Pflegeablauf insbesondere adressiert an den Server gesendet werden. Dies erfolgt insbesondere in Erwiderung auf das Erkennen einer entsprechenden Benutzereingabe, die das Senden anfordert. Alternativ kann das Senden erfolgen sobald eine Verbindung zum Server besteht. Vorzugsweise wird die Information über die Kommunikationsschnittstelle gesendet.

Nach einer ersten Variante umfassen die an den Server übertragenen Informationen sämtliche Informationen zu dem Ist-Pflegeablauf. Insbesondere ist aber vorgesehen, dass an den Server nur einen Ausschnitt mithin eine Teilmenge der Informationen zu dem Ist-Pflegeablauf übertragen wird. So werden insbesondere Informationen zu den Arbeitsschritten, die der Kategorie infektionsschutzrelevanter Arbeitsschritte zugeordnet sind, an den Server übertragenen. Es kann vorzugsweise vorgesehen sein, keine Informationen zu den Arbeitsschritten, die keiner Kategorie zugeordnet sind, an den Server übertragenen werden.

Der Server weist vorzugsweise eine Empfangseinrichtung zum Empfangen der Information zum Ist-Pflegeablauf auf. Die Empfangseinrichtung kann ausgebildet sein, die empfangene Information einem sendenden Pflegeunterstützungsgerät 100 zuzuordnen.

Der Server weist zudem eine Datenverarbeitungseinrichtung zum Auswerten der empfangenen Information, insbesondere unter Berücksichtigung der Identifikation des sendenden Pflegeunterstützungsgeräts 100 auf.

Die Datenverarbeitungseinrichtung ist vorzugsweise zur Konsolidierung der Informationen zum Ist-Pflegeablauf, die von einem oder verschiedenen Pflegeunterstützungsgeräten 100 in unterschiedlichen Beobachtungsvorgängen erfasst wurden, ausgebildet.

Der Server weist zudem eine Speichereinrichtung zum Speichern der Empfangenen Information oder des Ergebnisses der Auswertung auf. Der Speicher kann auch außerhalb des Servers angeordnet sein.

Der Server weist zudem eine Mensch Maschine Schnittstelle, beispielsweise einen Monitor und eine Computer Maus auf. Alternativ oder zusätzlich ist der Server mit einem Terminal verbunden, das eine Mensch Maschine Schnittstelle aufweist.

Durch die Mensch Maschine Schnittstelle kann die Oberfläche 500 bedient und angezeigt werden.

Besonders vorteilhaft ist es, den Server und das Pflegeunterstützungsgerät 100 so zu konfigurieren, dass eine Kommunikation oder die Übertragung der Information, insbesondere ohne weitere Benutzereingaben beziehungsweise automatisch erfolgen kann.

Die Verbindung zur Datenübertragung dazu kann beispielsweise direkt zwischen dem Server und dem Pflegeunterstützungsgerät 100 oder indirekt, beispielsweise über ein Kommunikationsnetzwerk oder mehrere Kommunikationsnetzwerke erfolgen. Die Verbindung kann drahtlos, drahtgebunden oder beides sein.

Das Pflegeunterstützungsgerät 100 und der Server bilden in einer Variante ein System zur Pflegeunterstützung. Dieses System ist besonders einfach bedienbar.

Nach einer weiteren Variante umfasst das System mehrere Pflegeunterstützungsgeräte 100. Das System kann auch mehrere Server oder Terminals umfassen.

## Patentansprüche

1. Pflegeunterstützungsgerät (100), umfassend
- eine Bildausgabeeinrichtung (102), die dazu eingerichtet ist, Symbole (201, ..., 212 ; 301, ..., 312 ; 401, ..., 412) darzustellen, die einen Pflegeablauf visualisieren,
- eine Vorgabeeinrichtung (103), die dazu eingerichtet ist, darzustellende Symbole basierend auf einem Soll-Pflegeablauf vorzugeben,
- eine Erfassungseinrichtung (104), die dazu eingerichtet ist, eine Benutzereingabe zu erfassen und dargestellte Symbole basierend auf der Benutzereingabe einem Ist-Pflegeablauf zuzuordnen,
- eine Bilderzeugungseinrichtung (105), die dazu eingerichtet ist, Symbole, die den Ist-Pflegeablauf visualisieren, zu erzeugen, wobei dazu eine Vergleichseinrichtung (106) eingerichtet ist, eine erste Reihenfolge, in der darzustellende Symbole basierend auf dem Soll-Pflegeablauf vorgegeben werden, mit einer zweiten Reihenfolge zu vergleichen, in der Benutzereingaben zu dargestellten Symbolen, die dem Ist-Pflegeablauf zugeordnet sind, erfasst wurden,
wobei die Bildausgabeeinrichtung (102) dazu eingerichtet ist, gleichzeitig die Symbole, die den Soll-Pflegeablauf visualisieren und die Symbole die den Ist-Pflegeablauf visualisieren, darzustellen, und
- eine Auswerteeinrichtung (107), die dazu eingerichtet ist, eine Spontanbewertung abhängig von einem Ergebnis eines Vergleichs der ersten Reihenfolge des Soll-Pflegeablaufs mit der zweiten Reihenfolge des Ist-Pflegeablaufs zu erzeugen und die dazu ausgebildet ist, bei fehlender Übereinstimmung der Stellen eines Arbeitsschritts in den beiden Reihenfolgen einem bestimmten dargestellten Symbol eine Spontanbewertung zuzuordnen,
wobei die Bildausgabeeinrichtung (102) dazu eingerichtet ist, die Spontanbewertung benachbart zu dem jeweiligen zugeordneten Symbol oder dem jeweiligen zugeordneten Symbol überlagert darzustellen.

2. Pflegeunterstützungsgerät (100) nach Anspruch 1, **dadurch gekennzeichnet, dass** die Bilderzeugungseinrichtung (102) eingerichtet ist, basierend auf der erfassten Benutzereingabe, mindestens ein dargestelltes Symbol, das dem Soll-Pflegeablauf zugeordnet ist, durch mindestens ein vorgegebenes Symbol, das dem Ist-Pflegeablauf zugeordnet ist, zu ersetzen, oder zu überlagern.

3. Pflegeunterstützungsgerät (100) nach einem der vorherigen Ansprüche, **gekennzeichnet durch** eine Eingabeeinrichtung (108), die dazu eingerichtet ist, basierend auf der erfassten Benutzereingabe einen Eingabedialog vorzugeben, wobei die Bildausgabeeinrichtung (102) den Eingabedialog gleichzeitig mit, insbesondere benachbart zu den Symbolen, die den Soll-Pflegeablauf und den Ist-Pflegeablauf visualisieren anzeigen kann.

4. Pflegeunterstützungsgerät (100) nach einem der vorherigen Ansprüche, **gekennzeichnet durch** eine Kommunikationsschnittstelle (109), die eingerichtet ist, Information über den Ist-Pflegeablauf insbesondere adressiert an einen Server zu senden.

5. Verfahren zur Pflegeunterstützung, umfassend
- Darstellen von Symbolen, die einen Pflegeablauf visualisieren, auf einer Bildausgabeeinrichtung (102),
- Vorgeben von darzustellenden Symbolen basierend auf einem Soll-Pflegeablauf,
- Erfassen einer Benutzereingabe,
- Zuordnen dargestellter Symbole zu einem Ist-Pflegeablauf basierend auf der Benutzereingabe,
- Erzeugen von Symbolen die den Ist-Pflegeablauf visualisieren, wobei eine erste Reihenfolge, in der darzustellende Symbole basierend auf dem Soll-Pflegeablauf vorgegeben werden, mit einer zweiten Reihenfolge verglichen wird, in der Benutzereingaben zu dargestellten Symbolen, die dem Ist-Pflegeablauf zugeordnet sind, erfasst wurden,
wobei auf der Bildausgabeeinrichtung (102) gleichzeitig Symbole, die den Soll-Pflegeablauf visualisieren, und Symbole die den Ist-Pflegeablauf visualisieren, dargestellt werden, und
Erzeugen einer Spontanbewertung mit einer Auswerteeinrichtung (107) abhängig von einem Ergebnis eines Vergleichs der ersten Reihenfolge des Soll-Pflegeablaufs mit der zweiten Reihenfolge des Ist-Pflegeablaufs welche bei fehlender Übereinstimmung der Stellen eines Arbeitsschritts in den beiden Reihenfolgen auftritt, und welche einem bestimmten dargestellten Symbol die Spontanbewertung zuordnet, wobei die Spontanbewertung benachbart zu dem jeweiligen zugeordneten Symbol oder dem jeweiligen zugeordneten Symbol überlagert dargestellt wird.

6. Verfahren nach Anspruch 5, **dadurch gekennzeichnet, dass** basierend auf der erfassten Benutzereingabe mindestens ein dargestelltes Symbol, das dem Soll-Pflegeablauf zugeordnet ist, durch mindestens ein vorgegebenes Symbol, das dem Ist-Pflegeablauf zugeordnet ist, ersetzt oder überlagert wird.

7. Verfahren nach einem der Ansprüche 5 oder 6, **gekennzeichnet durch**
- Vorgabe eines Eingabedialogs basierend auf der erfassten Benutzereingabe,
- Anzeigen des Eingabedialogs gleichzeitig mit, insbesondere benachbart zu den Symbolen, die den Soll-Pflegeablauf und den Ist-Pflegeablauf visualisieren.

8. Verfahren nach einem der Ansprüche 5 bis 7, **gekennzeichnet durch** Senden von Information über den Ist-Pflegeablauf insbesondere adressiert an einen Server.

9. System zur Pflegeunterstützung umfassend mindestens ein Pflegeunterstützungsgerät (100) nach Anspruch 4, und den Server, wobei der Server eine Empfangseinrichtung zum Empfangen der Information, und eine Mensch Maschine Schnittstelle zur Anzeige der Information, insbesondere auf einer Oberfläche (500), aufweist.

## Claims

1. A care support apparatus (100), comprising
- an image output device (102) which is configured to display symbols (201, ..., 212; 301, ..., 312; 401, ..., 412) which visualize a care procedure,
- a specification device (103) which is configured to specify symbols to be displayed on the basis of a target care procedure,
- a recording device (104) which is configured to record a user input and to assign displayed symbols to an actual care procedure on the basis of the user input,
- an image generating device (105) which is configured to generate symbols which visualize the actual care procedure, wherein a comparison device (106) is configured to compare a first sequence, in which the symbols to be displayed are specified on the basis of the target care procedure, with a second sequence, in which user inputs have been recorded as displayed symbols assigned to the actual care procedure,
wherein the image output device (102) is configured to display the symbols that visualize the target care procedure and the symbols that visualize the actual care procedure simultaneously, and
- an evaluation device (107) which is configured to generate a spontaneous evaluation depending on a result of a comparison of the first sequence of the target care procedure with the second sequence of the actual care procedure, and which is designed to assign a spontaneous evaluation to a specific displayed symbol in the absence of a correspondence between the positions of a working step in the two sequences,
wherein the image output device (102) is configured to display the spontaneous evaluation adjacent to the relevant assigned symbol or superimposed on the relevant assigned symbol.

2. The care support apparatus (100) according to claim 1, **characterized in that** the image generating device (102) is configured to replace or superimpose at least one displayed symbol assigned to the target care procedure with at least one predetermined symbol assigned to the actual care procedure on the basis of the recorded user input.

3. The care support apparatus (100) according to either of the preceding claims, **characterized by** an input device (108) which is configured to specify an input dialog on the basis of the recorded user input, the image output device (102) being able to display the input dialog simultaneously with, in particular adjacent to, the symbols which visualize the target care procedure and the actual care procedure.

4. The care support apparatus (100) according to any of the preceding claims, **characterized by** a communication interface (109) which is configured to transmit information about the actual care procedure, in particular addressed to a server.

5. A method for care support, comprising
- displaying symbols which visualize a care procedure on an image output device (102),
- specifying symbols to be displayed on the basis of a target care procedure,
- recording a user input,
- assigning displayed symbols to an actual care procedure on the basis of the user input,
- generating symbols which visualize the actual care procedure, wherein a first sequence, in which symbols to be displayed are specified on the basis of the target care sequence, is compared with a second sequence, in which user inputs have been recorded as displayed symbols assigned to the actual care procedure,
wherein symbols that visualize the target care procedure and symbols that visualize the actual care procedure are displayed simultaneously on the image output device (102), and
generating a spontaneous evaluation by means of an evaluation device (107) depending on a result of a comparison of the first sequence of the target care procedure with the second sequence of the actual care procedure, which evaluation occurs in the absence of a correspondence between the positions of a working step in the two sequences and which assigns the spontaneous evaluation to a specific displayed symbol, wherein the spontaneous evaluation is displayed adjacent to the relevant assigned symbol or superimposed on the relevant assigned symbol.

6. The method according to claim 5, **characterized in that** at least one displayed symbol assigned to the target care procedure is replaced or superimposed with at least one specified symbol assigned to the actual care procedure on the basis of the recorded user input.

7. The method according to claim 5 or claim 6, **characterized by**
- specifying an input dialog on the basis of the recorded user input,
- displaying the input dialog simultaneously with, in particular adjacent to, the symbols which visualize the target care procedure and the actual care procedure.

8. The method according to any of claims 5 to 7, **characterized by** transmitting information about the actual care procedure, in particular addressed to a server.

9. A system for care support comprising at least one care support apparatus (100) according to claim 4, and the server, wherein the server has a receiving device for receiving the information, and a human-machine interface for displaying the information, in particular on a surface (500).

## Revendications

1. Dispositif d'aide aux soins (100), comprenant
- un dispositif de sortie d'image (102) qui est configuré pour afficher des symboles (201, ..., 212; 301, ..., 312; 401, ..., 412) qui visualisent un déroulement de soins,
- un dispositif de spécification (103) qui est configuré pour spécifier des symboles à afficher sur la base d'un déroulement de soins cible,
- un dispositif de détection (104) qui est configuré pour détecter une entrée d'utilisateur et pour associer des symboles affichés à un déroulement de soins réel sur la base de l'entrée d'utilisateur,
- un dispositif de génération d'image (105) qui est configuré pour générer des symboles qui visualisent le déroulement de soins réel, dans lequel un dispositif de comparaison (106) est configuré pour comparer un premier ordre dans lequel des symboles à afficher sont spécifiés sur la base du déroulement de soins cible, à un deuxième ordre dans lequel on a détecté des entrées d'utilisateur quant à des symboles affichés qui sont associés au déroulement de soins réel,
dans lequel le dispositif de sortie d'image (102) est configuré pour afficher simultanément les symboles qui visualisent le déroulement de soins cible et les symboles qui visualisent le déroulement de soins réel, et
- un dispositif d'évaluation (107) qui est configuré pour générer une évaluation spontanée en fonction d'un résultat d'une comparaison du premier ordre du déroulement de soins cible au deuxième ordre du déroulement de soins réel et qui est conçu pour, s'il y a aucune correspondance entre les positions d'une étape de travail dans les deux ordres, associer une évaluation spontanée à un symbole affiché spécifique,
dans lequel le dispositif de sortie d'image (102) est configuré pour afficher l'évaluation spontanée de manière adjacente au symbole associé respectif ou de manière superposée au symbole associé respectif.

2. Dispositif d'aide aux soins (100) selon la revendication 1, **caractérisé par le fait que** le dispositif de génération d'image (102) est configuré pour remplacer, sur la base de l'entrée d'utilisateur détectée, au moins un symbole affiché qui est associé au déroulement de soins cible par au moins un symbole spécifié qui est associé au déroulement de soins réel, ou pour le superposer à celui-ci.

3. Dispositif d'aide aux soins (100) selon l'une quelconque des revendications précédentes, **caractérisé par** un dispositif d'entrée (108) qui est configuré pour spécifier, sur la base de l'entrée d'utilisateur détectée, un dialogue d'entrée, dans lequel le dispositif de sortie d'image (102) peut afficher ledit dialogue d'entrée simultanément avec les symboles qui visualisent le déroulement de soins cible et le déroulement de soins réel, en particulier de manière adjacente à ceux-ci.

4. Dispositif d'aide aux soins (100) selon l'une quelconque des revendications précédentes, **caractérisé par** une interface de communication (109) qui est configurée pour envoyer des informations sur le déroulement de soins réel, en particulier de manière adressée, à un serveur.

5. Procédé d'aide aux soins comprenant
- afficher des symboles qui visualisent un déroulement de soins, sur un dispositif de sortie d'image (102),
- spécifier des symboles à afficher sur la base d'un déroulement de soins cible,
- détecter une entrée d'utilisateur,
- associer des symboles affichés à un déroulement de soins réel sur la base de l'entrée d'utilisateur,
- générer des symboles qui visualisent le déroulement de soins réel, dans lequel un premier ordre dans lequel des symboles à afficher sont spécifiés sur la base du déroulement de soins cible est comparé à un deuxième ordre dans lequel des entrées d'utilisateur quant à des symboles affichés qui sont associés au déroulement de soins réel, ont été détectées,
dans lequel sur le dispositif de sortie d'image (102) sont affichés simultanément des symboles qui visualisent le déroulement de soins cible et des symboles qui visualisent le déroulement de soins réel, et
générer une évaluation spontanée au moyen d'un dispositif d'évaluation (107), en fonction d'un résultat d'une comparaison du premier ordre du déroulement de soins cible au deuxième ordre du déroulement de soins réel, qui apparaît s'il y a aucune correspondance entre les positions d'une étape de travail dans les deux ordres, et qui associe l'évaluation spontanée à un symbole spécifique affiché, dans lequel l'évaluation spontanée est affichée de manière adjacente au symbole associé respectif ou de manière superposée au symbole associé respectif.

6. Procédé selon la revendication 5, **caractérisé par le fait que**, sur la base de l'entrée d'utilisateur détectée, au moins un symbole affiché qui est associé au déroulement de soins cible est remplacé par au moins un symbole spécifié qui est associé au déroulement de soins réel, ou est superposé à celui-ci.

7. Procédé selon l'une quelconque des revendications 5 ou 6, **caractérisé par**
- spécifier un dialogue d'entrée sur la base de l'entrée d'utilisateur détectée,
- afficher le dialogue d'entrée simultanément avec les symboles qui visualisent le déroulement de soins cible et le déroulement de soins réel, en particulier de manière adjacente à ceux-ci.

8. Procédé selon l'une quelconque des revendications 5 à 7, **caractérisé par** l'envoi d'informations sur le déroulement de soins réel, en particulier de manière adressée, à un serveur.

9. Système d'aide aux soins comprenant au moins un dispositif d'aide aux soins (100) selon la revendication 4, et le serveur, dans lequel le serveur comprend un dispositif de réception destiné à recevoir l'information, et une interface homme-machine destinée à afficher l'information, en particulier sur une surface (500) .
